# EUROPEAN PATENT APPLICATION

(11) **EP 4 591 899 A1**
(43) Date of publication of application: **30.07.2025**
(21) Application number: 24305153.9
(22) Date of filing: 29.01.2024
(51) Int. Cl.: A61M 5/00, A61J 1/16

(54) **STACKABLE CONTAINER FOR TRANSPORTING MEDICAL DEVICES**

(71) Applicant: Becton Dickinson France, 38800 Le Pont-de-Claix (FR)
(72) Inventor: REMPFER, Simon, 38260 St Hilaire de la Cote (FR); LAVIGNE, Ferdinand, 38170 Seyssinet-Pariset (FR); ESNAULT, Gildas, 38100 Grenoble (FR); JACQUIER, Laurent, 38500 Saint Cassien (FR); Barcelo, Aurélie, 38400 Saint-Martin-d'Heres (FR)
(74) Representative: Germain Maureau

(57) **Abstract**

Provided herein is a tub for holding one or more nests of medical components. The tub includes a bottom wall, first and second pairs of opposed lateral sloped walls extending upwardly from the bottom wall and with corners formed between the respective lateral sloped walls, and a top flange formed on the lateral sloped walls, with the top flange defining an upper opening of the tub. Each of the first and second pairs of opposed lateral sloped walls comprises a stepped wall including a lower wall portion and an upper wall portion, with a ledge formed between the lower wall portion and the upper wall portion and including an inner surface and an outer surface. The tub is characterized in that includes ribs that enable stacking thereof with like tubs in a partially-nested arrangement, with the ribs provided at corners of the tub or in guide features of the tub.

## Description

### BACKGROUND OF THE INVENTION

### Field of the Invention

The present disclosure relates generally to nest arrangements for the packaging of medical containers (e.g., syringes) and/or components thereof (e.g., stoppers) and, more particularly, to an associated container, or tub, in which such nest arrangements are received.

### Description of Related Art

Medical containers and components for use with such containers, such as pre-fillable or prefilled syringes, cartridges, barrels, or stoppers, often need to be transported from one site to another site during or after manufacturing. For instance, medical containers or components thereof may be transported from a manufacturing site to a site at which the medical containers may be filled with a medicinal fluid. As used herein, a "medical fluid" can refer to a medication or another therapeutic agent used for treatment of chronic or acute conditions, as are known in the art. Exemplary therapeutic agents can include, for example, drugs, chemicals, biological, or biochemical substances that, when delivered in a therapeutically effective amount to the patient, achieve a desired therapeutic effect.

During transportation, the medical containers or components may be supported by a holding device, such as a tray or "nest." As known in the art, nests are available for holding/transporting syringe barrels or stoppers to be inserted in such syringe barrels (such as after pre-filling of a syringe barrel). One or more nests may be disposed within a holding and transport container, which may be referred to as a tub, with the tub having an opening allowing for placement of one or a plurality of nests therein and subsequent sealing thereof by a sealing cover.

With regard to tubs that are used for transporting a plurality of nests (and the medical containers or components held thereby), the tubs are typically manufactured and then shipped to a site where they are to be loaded with nests and the associated medical containers/components. While it may be desirable to stack a plurality of empty tubs (e.g., 3 tubs) for shipping in order to reduce an overall volume/space of the shipped tubs, it is recognized that existing tub designs make such stacking problematic. That is, with current tub designs, there is a high risk of tub deformation if the empty tubs are stacked together - with this being due to the low draft angles on the tub sidewalls and the likelihood that such draft angles will cause the tubs to stick together if they are packed in a stacked arrangement. Consequently, it is common for tubs to be transported in an alternating arrangement, i.e., top against top and bottom against bottom. It is recognized that such alternating of tub orientations when transporting leads to an increased shipping volume, with a large amount of wasted space in the packed tubs, therefore lowering the number of tubs that can be transported on/in standardized shipping containers, e.g., pallets, and leading to increased shipping costs.

Accordingly, a need exists in the art for a tub design that allows for the stacking of empty tubs during shipping, while minimizing the possibility of tub deformation resulting from such stacking. Allowing for such stacking while minimizing the possibility of tub deformation would provide for increased shipping efficiency, while also maintaining the structural integrity of the tubs.

### SUMMARY OF THE INVENTION

Provided herein is a tub for holding one or more nests each configured to retain a plurality of medical components. The tub includes a bottom wall, along with a first pair of opposed lateral sloped walls and a second pair of opposed lateral sloped walls extending upwardly from the bottom wall and defining an inner volume of the tub, with corners formed between the respective walls of the first pair of opposed lateral sloped walls and the second pair of opposed lateral sloped walls, and with the first pair of opposed lateral sloped walls, the second pair of opposed lateral sloped walls, and the corners including an inner wall surface and an outer wall surface. The tub also includes a top flange formed on the first pair of opposed lateral sloped walls and the second pair of opposed lateral sloped walls, with the top flange defining an upper opening of the tub. Each of the first pair of opposed lateral sloped walls and the second pair of opposed lateral sloped walls comprises a stepped wall including a lower wall portion and an upper wall portion, with a ledge formed between the lower wall portion and the upper wall portion and including an inner surface and an outer surface. The tub is characterized in that each corner comprises a corner rib extending inwardly from the inner surface thereof, with each corner rib extending vertically upwardly from the ledge to a location that is vertically below the top flange.

In accordance with some aspects of the disclosure, the corner ribs provide for stacking of the tub with another tub of identical construction, the tubs comprising a lower tub and an upper tub, with a top edge of each of the corner ribs on the lower tub engaging the outer surface of the ledge on the upper tub, to support the upper tub on the lower tub and partially nest the upper tub within the lower tub.

In accordance with some aspects of the disclosure, the ledge extends outwardly from a top edge of the lower wall portion to a bottom edge of the upper wall portion, with the corner guides extending upwardly from the inner surface of ledge.

In accordance with some aspects of the disclosure, the ledge is parallel to the bottom wall and is configured to support a bottom-most nest of the one or more nests held in the tub.

In accordance with some aspects of the disclosure, each of the first pair of opposed lateral sloped walls, each of the second pair of opposed lateral sloped walls, or both each of the first pair of opposed lateral sloped walls and each of the second pair of opposed lateral sloped walls, includes one or more guide features thereon extending upwardly from the ledge, with each of the one or more guide features defining a projection on the inner wall surface and a recess on the outer wall surface.

In accordance with some aspects of the disclosure, each of the guide features includes a chamfered top surface that is inset inwardly and downwardly from the top flange.

In accordance with some aspects of the disclosure, the recess of each respective guide feature of the upper tub slides down into engagement with the projection of each respective guide feature of the lower tub, to guide the upper tub into stacking engagement with the lower tub.

In accordance with some aspects of the disclosure, with the upper tub partially nested within the lower tub, a height of the stacked upper tub and lower tub is approximately 1. 5x a height of one of the upper tub or the lower tub.

In accordance with some aspects of the disclosure, the tub is configured to hold the one or more nests therein, with each of the one or more nests comprising a plurality of chimneys configured to retain syringe stoppers therein.

Also provided herein is a tub for holding one or more nests each configured to retain a plurality of medical components. The tub includes a bottom wall, along with a first pair of opposed lateral sloped walls and a second pair of opposed lateral sloped walls extending upwardly from the bottom wall and defining an inner volume of the tub, with the first pair of opposed lateral sloped walls and the second pair of opposed lateral sloped walls including an inner wall surface and an outer wall surface. The tub also includes a top flange formed on the first pair of opposed lateral sloped walls and the second pair of opposed lateral sloped walls, with the top flange defining an upper opening of the tub. Each of the first pair of opposed lateral sloped walls and the second pair of opposed lateral sloped walls comprises a stepped wall including a lower wall portion and an upper wall portion, with a ledge formed between the lower wall portion and the upper wall portion. Each of the first pair of opposed lateral sloped walls, each of the second pair of opposed lateral sloped walls, or both each of the first pair of opposed lateral sloped walls and each of the second pair of opposed lateral sloped walls, includes one or more guide features thereon extending upwardly from the ledge, with each of the one or more guide features defining a projection on the inner wall surface and a recess on the outer wall surface. The tub is characterized in that each of the one or more guide features includes a rib positioned within the recess that extends from a top end of the recess to a bottom end of the recess.

In accordance with some aspects of the disclosure, the guide features provide for stacking of the tub with another tub of identical construction, the tub comprising an upper tub and the another tub comprising a lower tub, with a bottom edge of each of the ribs on the upper tub engaging the lower tub, to support the upper tub on the lower tub and partially nest the upper tub with the lower tub.

In accordance with some aspects of the disclosure, the bottom edge of each of the ribs on the upper tub engages a top surface of a respective projection on the lower tub, to support the upper tub on the lower tub and partially nest the upper tub with the lower tub.

In accordance with some aspects of the disclosure, the projection of each respective guide pillar extends up to the top flange, with the top surface comprising a flat surface extending inwardly from the top flange.

In accordance with some aspects of the disclosure, the top surface of each respective guide pillar comprises a chamfered top surface that is inset inwardly and downwardly from the top flange.

In accordance with some aspects of the disclosure, the chamfered top surface comprises an angled top surface that slopes inwardly and downwardly, and wherein the bottom edge of each of the ribs comprises an angled bottom surface that mates with the angled top surface.

### BRIEF DESCRIPTION OF THE DRAWINGS

FIG. 1 is a perspective view of a packaging system for storing and shipping a plurality of medical components, according to an embodiment of the disclosure;
FIG. 2 is an exploded view of the packaging system of FIG. 1;
FIG. 3 is a top perspective view of a tub that may be included in the packaging system of FIG. 1, according to an embodiment of the disclosure;
FIG. 4 is a bottom perspective view of the tub of FIG. 3;
FIG. 5 is a perspective view showing the tub of FIG. 3 stacked with another tub of identical construction, with the tubs stackable when in an empty state;
FIG. 6 is a cross-sectional view taken along line 5-5 of FIG. 5;
FIG. 7 is a top perspective view of a tub that may be included in the packaging system of FIG. 1, according to an embodiment of the disclosure;
FIG. 8 is a bottom perspective view of the tub of FIG. 7;
FIG. 9 is a detailed view of Section A of FIG. 8, illustrating a rib included in a guide feature of the tub;
FIG. 10 is a perspective view showing the tub of FIG. 7 stacked with another tub of identical construction, with the tubs stackable when in an empty state;
FIG. 11 is a cross-sectional view taken along line 10-10 of FIG. 10, illustrating a chamfer feature of the tubs, according to an embodiment of the disclosure;
FIG. 12 is a cross-sectional view taken along line 10-10 of FIG. 10, illustrating a chamfer feature of the tubs, according to another embodiment of the disclosure;
FIG. 13 is a top perspective view of a tub that may be included in the packaging system of FIG. 1, according to an embodiment of the disclosure;
FIG. 14 is a bottom perspective view of the tub of FIG. 13;
FIG. 15 is a detailed view of Section B of FIG. 14, illustrating a rib included in a guide feature of the tub;
FIG. 16 is a perspective view showing the tub of FIG. 13 stacked with another tub of identical construction, with the tubs stackable when in an empty state; and
FIG. 17 is a cross-sectional view taken along line 16-16 of FIG. 16.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

Aspects and embodiments of the disclosure are directed to tubs that are used for transporting a plurality of nests and the medical containers or components held thereby. The tubs are designed so as to provide for the nested stacking of empty tubs during shipping, while minimizing the possibility of tub deformation resulting from such nested stacking.

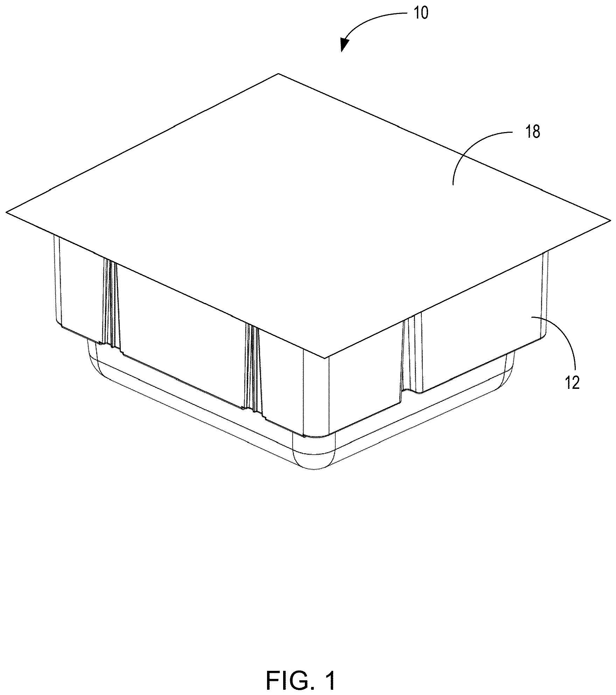

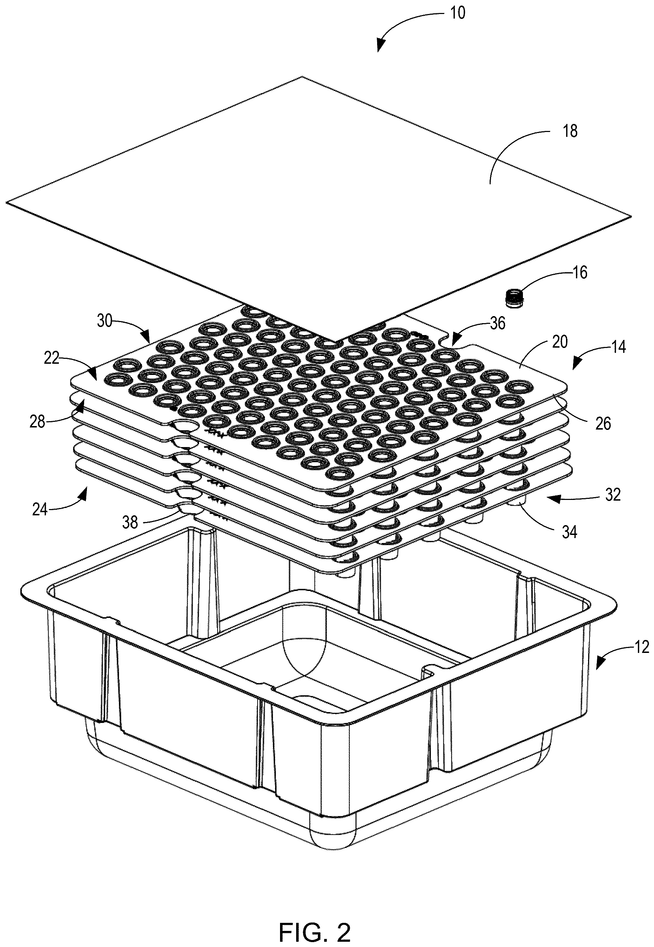

Referring to FIGS. 1 and 2, shown is a non-limiting embodiment of a packaging system 10 that may be used for the packaging and transporting of a plurality of medical components therein. The packaging system 10 includes a container or tub 12 in which a plurality of nests 14 may be housed, with each nest 14 retaining a plurality of medical components therein. In the illustrated embodiment, each of nests 14 is configured to retain therein a plurality of stoppers 16 that may be utilized in syringe containers (e.g., prefilled syringes), as known in the art, although it is recognized that nests 14 could alternatively retain other medical containers or components therein. The packaging system 10 also includes a sealing cover 18 that may be applied over the tub 12 after the nests 14 are packaged therein, to provide a sterile environment for storing and shipping the stoppers 16.

Exemplary nests 14 that may be retained in tub 12 are shown in FIG. 2. The nests 14 each comprise a support plate 20 having a first or upper surface 22, a second or lower surface 24, and a peripheral edge 26 extending about the support plate 20 between the upper surface 22 and the lower surface 24. In some examples, the perimeter of support plate 20 is substantially rectangular in shape - having longitudinal sides 28 and transverse sides 30. In some embodiments, the longitudinal sides 26 may be longer than the transverse sides 30. In other embodiments, the support plate 20 may be square, in which case the longitudinal sides 28 and the transverse sides 30 are the same length.

The nests 14 further comprise tubular walls, members, or ridges - referred to hereafter as "chimneys 32" - that protrude outward and downward from a plane defined by the upper surface 22 of the support plate 20. Each chimney 32 can include a wall 34 (i.e., a cylindrical external wall and/or frustoconical internal wall) that defines a receptacle 35 configured to receive a stopper 16 therein, with the receptacle sized to retain an entirety of the stopper 16. The receptacle 35 includes a top opening at a top end of the chimney 32 and a bottom opening at a bottom end of the chimney 32. As shown in FIG. 2, according to some aspects of the disclosure, the nests 14 can be configured to include 100 chimneys 32 loaded with stoppers 16 for use with 1-3 mL syringes. Alternatively, the nests 14 can be configured to include 160 chimneys 32 loaded with stoppers 16 for use with 1 mL syringes, or another number of chimneys 32 that is dependent on the size of the stoppers 16 retained therein.

In some embodiments, the nests 14 may further include therein median finger notches 36 on opposing longitudinal sides 28 (or transverse sides 30) of the plate 20, with each of the finger notches 36 including a delimiting wall 38. The finger notches 36 allow the insertion of a user's fingers therethrough, for catching the nest 14, and the delimiting walls 38 provide increased contact surfaces for these fingers.

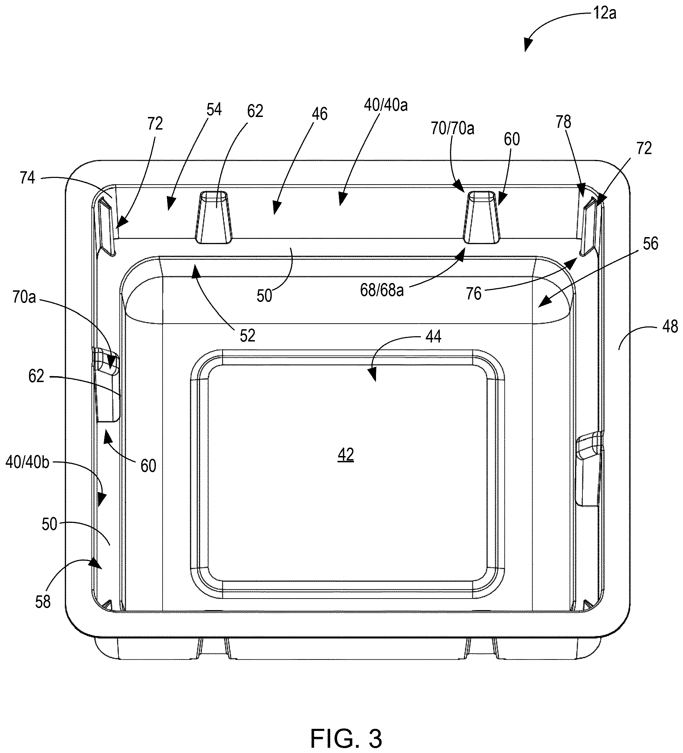

With reference now to FIGS. 3-6, a tub 12a that may be included in the packaging system 10 is shown and described in greater detail, in accordance with one embodiment of the disclosure. The tub 12a is formed by a single part of molded plastic material (e.g., an injection molded polystyrene tub, as a non-limiting example) and comprises a plurality of sidewalls 40 - including first opposed lateral sidewalls 40a and second opposed lateral sidewalls 40b - that extend upwardly from a bottom wall 42 that defines a base of the tub 12a. Each of the sidewalls 40 may be configured as a sloped sidewall that does not extend exactly vertical from bottom wall 42, but instead extends up at an angle therefrom. The interior volume of the tub 12a is hollow and is defined by an interior surface 44 of the bottom wall 42 and an interior surface 46 of the sidewalls 40.

Opposite the bottom wall 42, the tub 12a is open such that the nests 12 may be inserted therein. A top flange 48 may be provided at a top end of the sidewalls 40 opposite to which the bottom wall 42 is located. The top flange 48 is configured to provide for coupling of sealing cover 18 (FIGS. 1 and 2) thereto (after one or more nests 14 having stoppers 16 contained therein are disposed in the tub 12a).

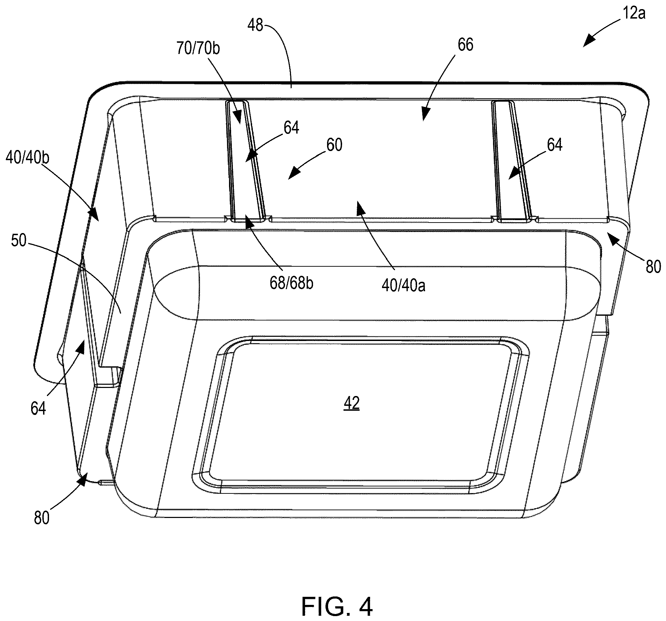

As illustrated in FIGS. 3 and 4, each of the sidewalls 40 may have a stepped construction that defines a ledge 50 positioned between a bottom sidewall portion 52 and a top sidewall portion 54 of each sidewall 40. The top sidewall portion 54 is offset outwardly from the bottom sidewall portion 52, such that an interior opening 56 defined by a perimeter of the ledge 50 has a smaller area than the opening 58 of the tub 12a defined by the flange 48. In use of the tub 12a, the ledge 50 provides a horizontal surface configured to support the plate 20 of a bottom-most nest 14 (FIG. 2) such that, when the plate 20 is disposed in the tub 12a, the lower surface 24 of the plate 20 is disposed on the ledge 50. While the ledge 50 is shown as extending continuously about a perimeter of the interior surface 46 of sidewalls 40, it is recognized that ledge 50 could also be formed discontinuously about a perimeter of the interior surface 46 of sidewalls 40.

According to aspects of the disclosure, the tub 12a includes a plurality of features thereon that provide for stacking of the tub 12a with another tub of identical construction. In particular, the tub 12a includes features thereon that provide for the stacking of multiple tubs 12a when in an empty state (i.e., when not in use), such as when the tubs 12a are shipped from a facility where they are manufactured to a location where they are to be loaded with nests 14. As explained in further detail below, the construction of tub 12a enables the stacking of multiple tubs 12a (e.g., 3 tubs) together during shipping, while minimizing the possibility of tub deformation resulting from such stacking - as may occur if the tubs 12a are fully nested within each other due to the outwardly exerted force applied on the sloped sidewalls 40 thereof.

As shown in FIGS. 3 and 4 at least some of the sidewalls 40 include guide features 60 thereon that provide for alignment and engaging of multiple tubs 12a when it is desired to stack tubs together. In the illustrated embodiment, both first opposed lateral sidewalls 40a and second opposed lateral sidewalls 40b are shown as including guide features 60, although it is recognized that only first opposed lateral sidewalls 40a or second opposed lateral sidewalls 40b may include guide features 60 thereon, according to other embodiments of the disclosure. Additionally, in the illustrated embodiment, first opposed lateral sidewalls 40a are shown as each including two guide features 60 thereon, with the guide features 60 aligned in identical positions on each of the lateral sidewalls 40a, while the second opposed lateral sidewalls 40b are shown as each including a single guide feature, with the guide features 60 offset from one another on the opposing lateral sidewalls 40b.

The guide features 60 are formed so as to appear as projections 62 that extend inwardly from the interior surface 46 of the sidewalls 40 and appear as recesses 64 depressed inwardly into an exterior surface 66 of the sidewalls 40. Each guide feature 60 extends vertically upwardly from a bottom end 68 thereof that is joined with ledge 50 to a top end 70 thereof vertically located just below the top flange (and opening) at the top end of the sidewalls 40. The guide features 60 are formed so as to taper from the bottom end 68 to the top end 70 - i.e., the projections 62 taper from their bottom end 68a to their top end 70a and the recesses 64 taper from their bottom end 68b to their top end 70b.

According to some embodiments, the top end or surface 70a of each projection 62 of guide features 60 may be configured as what is referred to herein as a "chamfered" top surface. The chamfered top surface 70a of the projection 62 is inset inwardly and downwardly from the top flange 48 of tub 12a. In some embodiments, and as shown in FIG. 3, the chamfered top surface 70a of the projection 62 is formed as a sloped surface that is angled inwardly and downwardly.

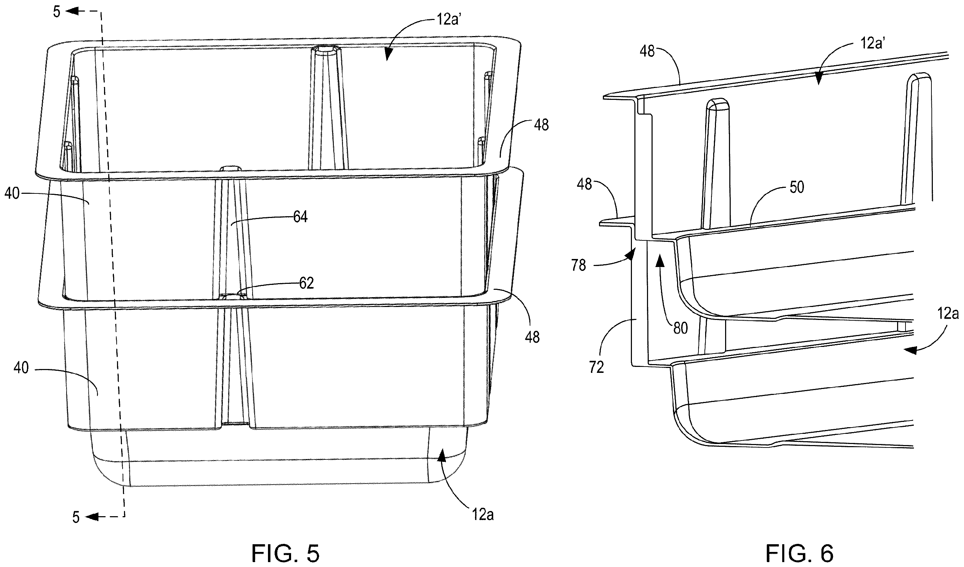

As shown in FIG. 5, the guide features 60 may function to align multiple tubs 12a when desired to stack such tubs 12a in a nested arrangement. That is, in a first relative position of an upper tub 12a above a lower tub 12a', the recesses 64 of the upper tub 12 are in coincidence with the projections 62 of the lower tub 12a', and therefore the projections 62 of the lower tub 12a' are able to be slidingly received in the recesses 64 of the upper tub 12a as the upper tub is lowered down onto the lower tub - thus allowing a nesting of said upper tub 12a in said lower tub 12a'.

According to aspects of the disclosure, the tub 12a also includes thereon a plurality of ribs 72 that control the amount/depth by which tubs 12a, 12a' nest together when stacked. In particular, in each corner 74 of the tub 12a between adjacent sidewalls 40, a rib 72 may be provided that protrudes inwardly from the interior surface 46 of the corner 74 (i.e., interior surface 46 of sidewalls 40). In some embodiments, each corner rib 72 may extend orthogonally inward from the interior surface 46 of the corner 74, with each of the corners 74 formed as a radiused corner between adjacent sidewalls 40. Each corner rib 72 extends vertically upwardly from a bottom end 76 thereof that is joined with ledge 50 to a top end 78 thereof vertically located below the top flange 48 (and opening) at the top end of the sidewalls 40. According to embodiments, the top edge or surface 78 of each corner rib 72 is formed as a flat surface that is in parallel with the bottom wall 42 of the tub 12a.

With the ribs 72 positioned in each of the corners of tub 12a as described above, and with the top surface 78 of each corner rib 72 oriented as described above, the ribs 72 are configured to provide a support surface for another tub 12a' that is to be stacked onto a lower tub 12a. That is, as shown in FIG. 5 and in further detail in FIG. 6, when an upper tub 12a' is to be stacked onto (and nested with) a lower tub 12a, the corner ribs 72 of the lower tub 12a are arranged and configured such that they will engage and support the upper tub 12a' when it is lowered down onto the lower tub 12a. In particular, the top surface 78 of corner ribs 72 will abut and come into engagement with an exterior surface 80 of the ledge 50 of the upper tub 12a'. With the corner ribs 72 of lower tub 12a engaging and supporting the upper tub 12a' in this manner, the amount/depth by which tubs 12a, 12a' nest together when stacked is controlled. As a non-limiting example, for tubs 12a, 12a' that are 100 mm in height, the upper tub 12a' may be nested within the lower tub 12a such that the upper tub 12a' extends outwardly and upwardly from the lower tub 12a by between 50-60mm, and preferably by 57.5mm - with 42.5mm of the upper tub 12a' thus received within the interior volume of the lower tub 12a (at a vertical level below the top flange 48 of lower tub 12a). With the stacking and nesting of the tubs 12a, 12a' controlled by corner ribs 72 as described above, the tubs 12a, 12a' may be nested together in a desired manner when in an empty state, while minimizing the possibility of tub deformation resulting from such stacking.

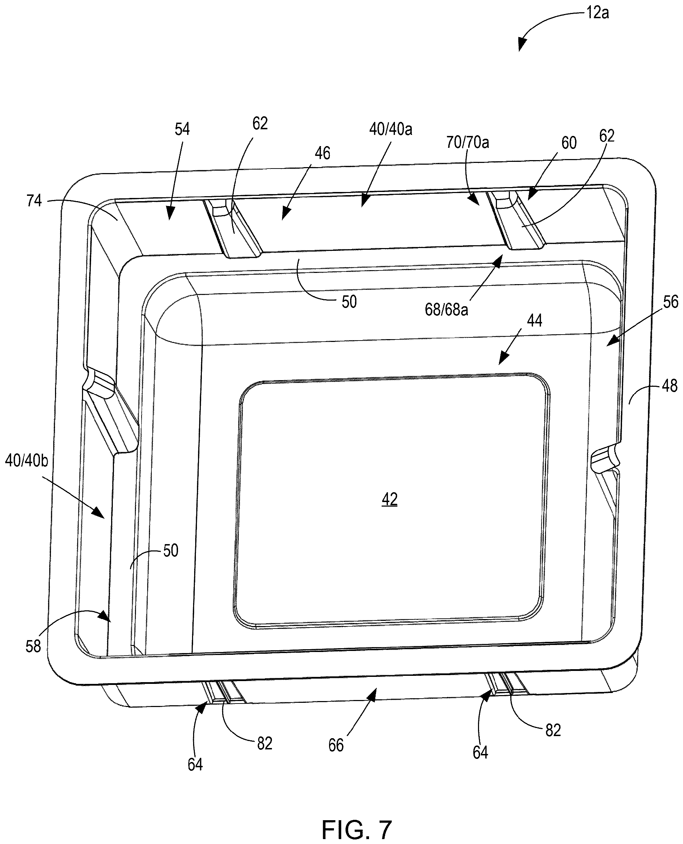

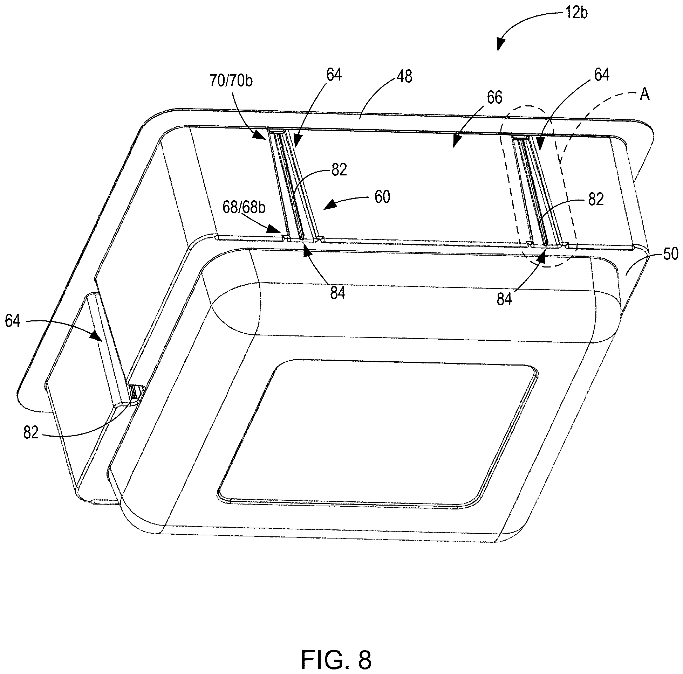

Referring now to FIGS. 7-12, a tub 12b is shown according to another embodiment of the disclosure. The structure of tub 12b is substantially similar to the structure of tub 12a, and thus common features between tubs 12a, 12b are numbered identically. In general, tub 12b includes sidewalls 40, bottom wall 42, and top flange 48 - with sidewalls 40 having a stepped construction that defines a ledge 50 positioned between a bottom sidewall portion 52 and a top sidewall portion 54 of each sidewall 40. The tub 12b also includes guide features 60 thereon that provide for alignment and engaging of multiple tubs when it is desired to stack tubs together. In the illustrated embodiment, both first opposed lateral sidewalls 40a and second opposed lateral sidewalls 40b are shown as including guide features 60 - with the first opposed lateral sidewalls 40a including two guide features 60 thereon and the second opposed lateral sidewalls 40b including a single guide feature thereon.

As described above, the guide features 60 are formed so as to appear as projections 62 that extend inwardly from the interior surface 46 of the sidewalls and appear as recesses 64 depressed inwardly into an exterior surface 66 of the sidewalls 40. Each guide feature 60 extends vertically upwardly from a bottom end 68a thereof that is joined with ledge 50 to a top end 70 thereof vertically located just below the top flange 48 (and opening) at the top end of the sidewalls 40. According to some embodiments, the top end or surface 70a of each projection 62 of guide features 60 may be configured as a chamfered top surface. The chamfered top surface 70a of the projection 62 is inset inwardly and downwardly from the top flange 48 of tub 12b.

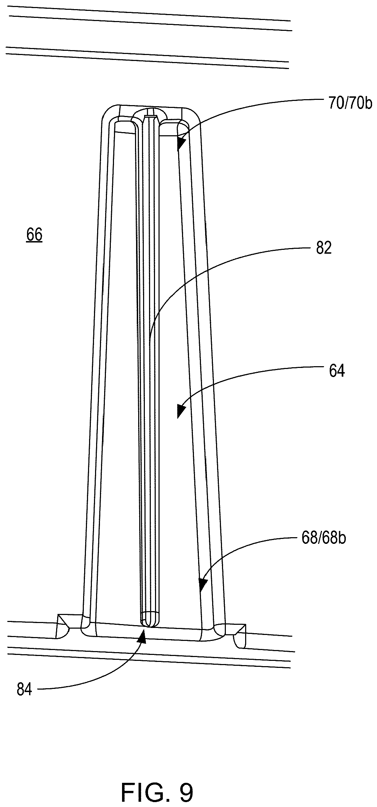

According to aspects of the disclosure, the tub 12b also includes thereon a plurality of ribs 82 that control the amount/depth by which tubs 12b nest together when stacked. However, different from the tub 12a where the ribs 72 are provided in each corner 74 of the tub 12a on the interior surface 46 of the corner (i.e., interior surface 46 of sidewalls 40), the ribs 82 in tub 12b are provided as part of the guide features 60. In particular, each guide feature 60 includes a rib 82 that is formed within the recess 64 thereof on the exterior surface 66 of sidewalls 40. As best shown in FIG. 9, each rib 82 is aligned vertically within the recess 64 and may be centered horizontally within the recess 64. In some embodiments, the rib 82 runs a full length of the recess 64 - from a closed top end 70b of the recess 64 to an open bottom end 68b of the recess 64, while in other embodiments the rib 82 may run less than a full length of the recess 64 (i.e., rib 82 does not extend to the bottom end 68b of recess 64 - with it recognized that a length of the ribs 82 controls a stacking depth of tubs. Furthermore, each rib 82 extends orthogonally outward from the exterior surface 66 of the sidewall within recess 64. According to embodiments, the amount by which the ribs 82 protrudes outwardly from the exterior surface 66 of the sidewall 40 within recess 64 may be an amount that is less than or equal to a depth of the recess 64.

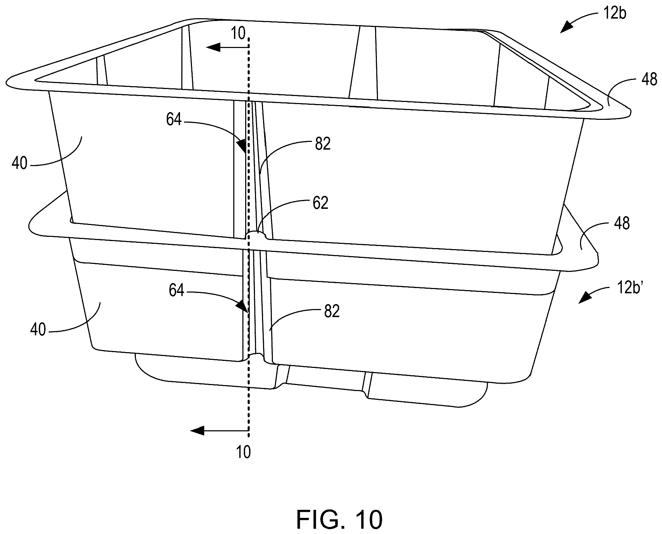

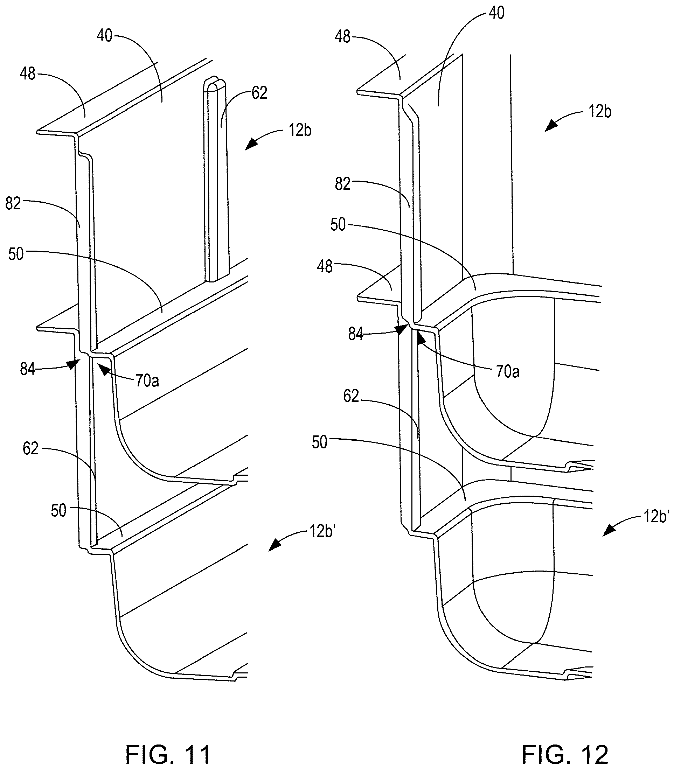

With the ribs 82 formed on guide features 60 of tub 12b as described above, the ribs 82 of tub 12b are configured to engage with corresponding guide features 60 of another tub 12b' onto which the tub 12b is to be stacked - i.e., tub 12b is an upper tub to be stacked on another tub 12b' is a lower tub. That is, as shown in FIG. 10 and in further detail in FIGS. 11 and 12, when an upper tub 12b is to be stacked onto (and nested with) a lower tub 12b', the ribs 82 of the upper tub 12b are arranged and configured such that a lower edge 84 thereof will engage a top surface 70a of the projections 62 on lower tub 12b', with engagement of the lower edges 84 of the ribs 82 and the top surface 70a of the projections 62 supporting the upper tub 12b when it is lowered down onto the lower tub 12b'. With the top surface 70a of projections 62 being a chamfered top surface that is inset inwardly and downwardly from the top flange 48 of tub 12b, the bottom edge 84 of the ribs 82 of the upper tub 12b thus engage the chamfered top surface 70a of the projections 62 of the lower tub 12b' at a location that is at a vertical level below the top flange 48 of lower tub 12b'.

According to aspects of the disclosure, the structure of the ribs 82 and the projections 62 of guide features 60 can vary to determine the exact engagement between the bottom edge 84 of the ribs 82 of the upper tub 12b engaging the chamfered top surface 70a of the projections 62 of the lower tub 12b'. In one embodiment, and as shown in FIG. 11, the bottom edge 84 of the ribs 82 of the upper tub 12b and the chamfered top surface 70a of the projections 62 of the lower tub 12b' are each formed as flat (horizontal) surfaces that provide for engagement therebetween. In another embodiment, and as shown in FIG. 12, the bottom edge 84 of the ribs 82 of the upper tub 12b and the chamfered top surface 70a of the projections 62 of the lower tub 12b' are each formed as angled surfaces that provide for engagement therebetween.

With the bottom edge 84 of the ribs 82 of the upper tub 12b engaging the chamfered top surface 70a of the projections 62 of the lower tub 12b' in the manner described above, the amount/depth by which tubs 12b, 12b' nest together when stacked is controlled. In general, the stacked/nested tubs 12b, 12b' will have an overall height that is less than a height of two separated tubs 12b, 12b' that are not nested with each other - with a height of the nested tubs 12b, 12b' being approximately 1.5x the height of a single tub 12b, 12b' (due to a portion of the upper tub 12b being received within the interior volume of the lower tub 12b', at a vertical level below the top flange 48 of lower tub). With the stacking and nesting of the tubs 12b, 12b' controlled by the ribs 82 on the guide features 60 as described above, the tubs 12b, 12b' may be nested together in a desired manner when in an empty state, while minimizing the possibility of tub deformation resulting from such stacking.

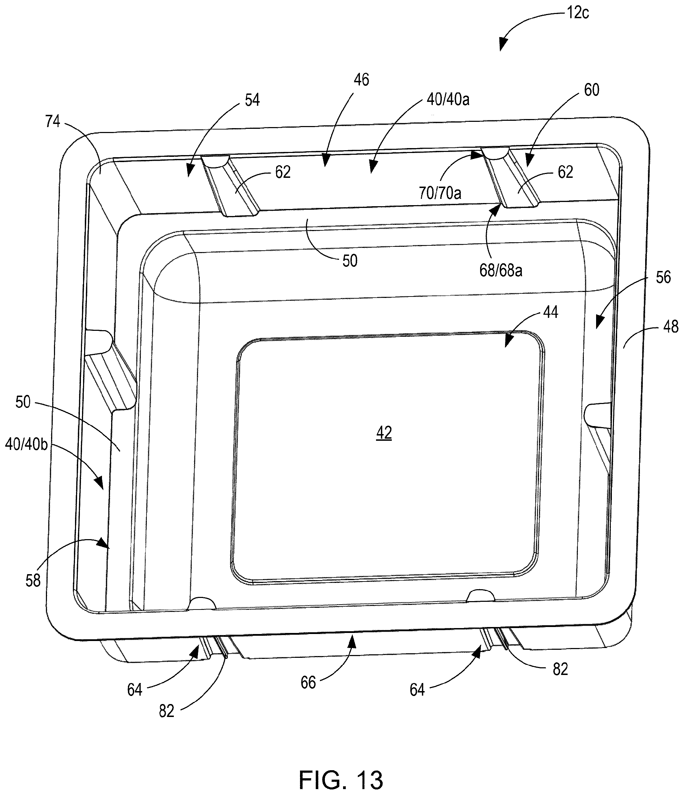

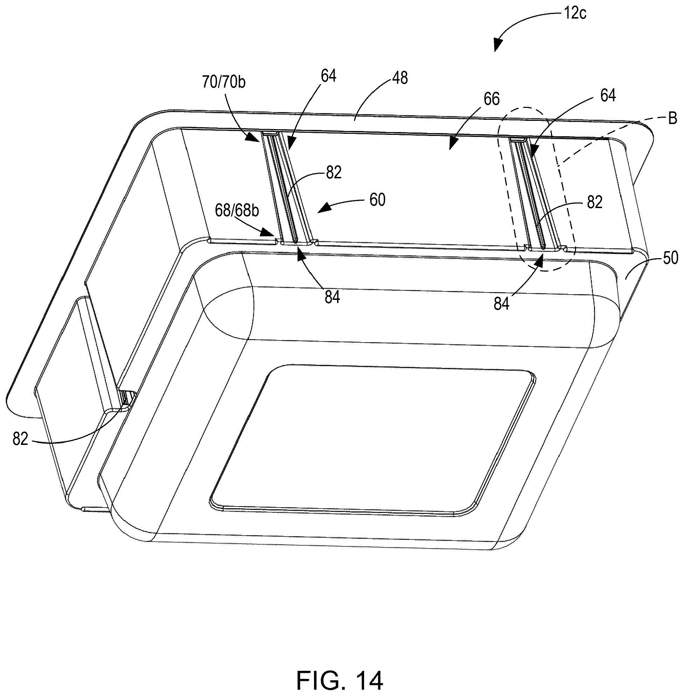

Referring now to FIGS. 13-17, a tub 12c is shown according to another embodiment of the disclosure. The structure of tub 12c is substantially similar to the structure of tubs 12a, 12b and thus common features between tubs 12a, 12b, 12c are numbered identically. In general, tub 12c includes sidewalls 40, bottom wall 42, and top flange 48 - with sidewalls 40 having a stepped construction that defines a ledge 50 positioned between a bottom sidewall portion 52 and a top sidewall portion 54 of each sidewall 40. The tub 12c also includes guide features 60 thereon that provide for alignment and engaging of multiple tubs when it is desired to stack tubs together. In the illustrated embodiment, both first opposed lateral sidewalls 40a and second opposed lateral sidewalls 40b are shown as including guide features 60 - with the first opposed lateral sidewalls 40a including two guide features 60 thereon and the second opposed lateral sidewalls 40b including a single guide feature 60 thereon.

As described above, the guide features 60 are formed so as to appear as projections 62 that extend inwardly from the interior surface 46 of the sidewalls and appear as recesses 64 depressed inwardly into an exterior surface 66 of the sidewalls 40. However, different from on tubs 12a, 12b, each guide feature 60 of tub 12c extends vertically upwardly from a bottom end 68 thereof that is joined with ledge 50 to a top end 70 thereof that is vertically aligned with the top end of the sidewalls 40 and with the top flange 48 (and opening). The top end or surface 70a of each projection 62 of guide features 60 is formed as a flat (horizontal) surface that is thus planar with the top flange 48.

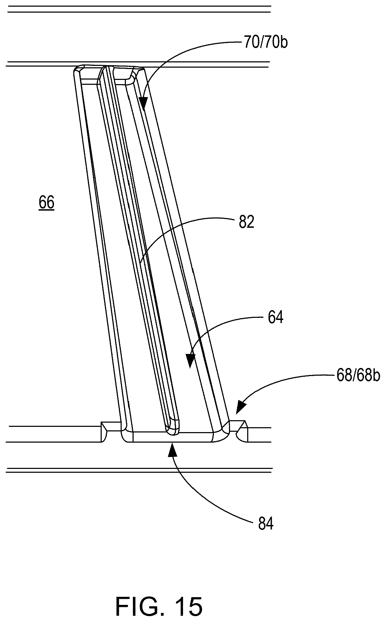

According to aspects of the disclosure, the tub 12c also includes thereon a plurality of ribs 82 that control the amount/depth by which tubs 12c nest together when stacked. As described above for tub 12b, the ribs 82 in tub 12c are provided as part of the guide features 60 - with each guide feature 60 including a rib 82 that is formed within the recess 64 thereof on the exterior surface 66 of sidewalls 40. As best shown in FIG. 15, each rib 82 is aligned vertically within the recess 64 and may be centered horizontally within the recess 64. In some embodiments, each rib 82 runs a full length of the recess 64 - from a closed top end 70b of the recess 64 to an open bottom end 68b of the recess 64, while in other embodiments the rib 82 may run less than a full length of the recess 64 (i.e., rib 82 does not extend to the bottom end 68b of recess 64 - with it recognized that a length of the ribs 82 controls a stacking depth of tubs. Furthermore, each rib 82 extends orthogonally outward from the exterior surface 66 of the sidewall 40 within recess 64. According to embodiments, the amount by which the rib 82 protrudes outwardly from the exterior surface 66 of the sidewall 40 within recess 64 by an amount that is equal to a depth of the recess 64.

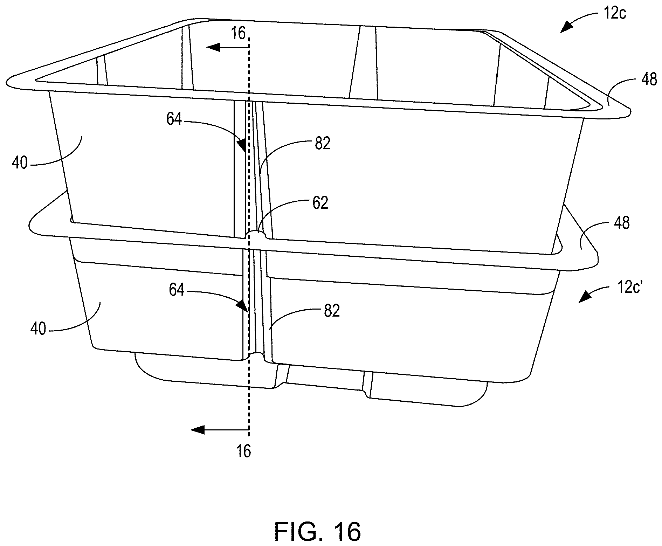

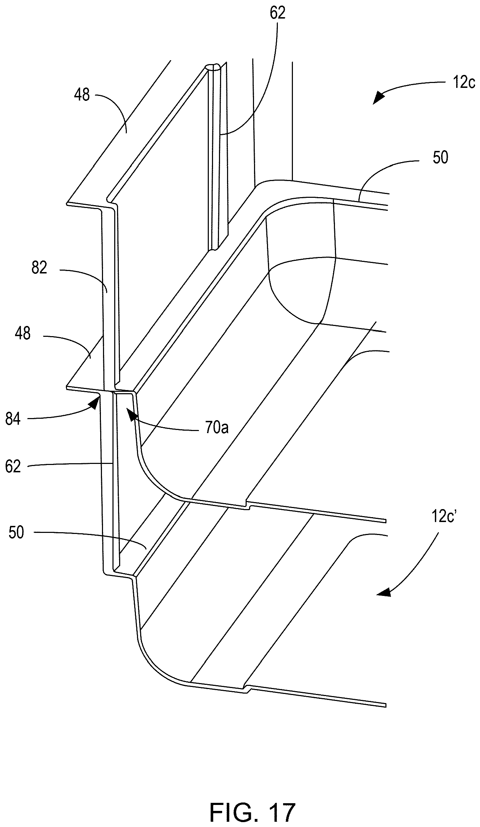

With the ribs 82 formed on guide features 60 of tub 12c as described above, the ribs 82 of tub 12c are configured to engage with corresponding guide features 60 of another tub 12c' onto which the tub 12c is to be stacked - i.e., tub 12c is an upper tub to be stacked on another lower tub 12c'. That is, as shown in FIG. 16 and in further detail in FIG. 17, when an upper tub 12c is to be stacked onto (and nested with) a lower tub 12c', the ribs 82 of the upper tub 12c are arranged and configured such that a lower edge 84 thereof will engage a top surface of the projections 62 on lower tub 12c', with engagement of the lower edges of the ribs 82 and the top surface of the projections 62 supporting the upper tub 12c when it is lowered down onto the lower tub 12c'. With the top surface 70a of projections 62 being a flat top surface that is vertically aligned with the top flange 48 of tub 12c, the bottom edge 84 of the ribs 82 of the upper tub 12c thus engage the top surface 70a of the projections 62 of the lower tub 12c' at a location that is vertically aligned with the top flange 48 of lower tub 12c'.

With the bottom edge 84 of the ribs 82 of the upper tub 12c engaging the top surface of the projections 62 of the lower tub 12c' in the manner described above, the amount/depth by which tubs nest together when stacked is controlled. As a non-limiting example, for tubs 12c, 12c' that are 100 mm in height, the upper tub 12c may be nested within the lower tub 12c' such that the upper tub 12c extends outwardly and upwardly from the lower tub 12c' by between 60-70mm, and preferably by 65.0mm - with 35.0mm of the upper tub 12c thus received within the interior volume of the lower tub 12c' (at a vertical level below the top flange 48 of lower tub 12c'). With the stacking and nesting of the tubs 12c, 12c' controlled by the ribs 82 on the guide features 60 as described above, the tubs 12c, 12c' may be nested together in a desired manner when in an empty state, while minimizing the possibility of tub deformation resulting from such stacking.

Beneficially, embodiments of the invention thus provide tubs that are used for transporting a plurality of nests and the medical containers or components held thereby. The tubs are designed so as to provide for the nested stacking of empty tubs during shipping, while minimizing the possibility of tub deformation resulting from such nested stacking. The tubs include ribs formed thereon that control the depth at which an upper tub is nested with a lower tub during stacking, so as to control the forces applied to the tub sidewalls of the lower tub(s). Structural integrity of the tubs may thus be maintained, while still providing for improved shipping efficiency of the tubs by shipping them in a nested arrangement.

Although the present disclosure has been described in detail for the purpose of illustration based on what is currently considered to be the most practical and preferred embodiments or aspects, it is to be understood that such detail is solely for that purpose and that the present disclosure is not limited to the disclosed embodiments or aspects, but, on the contrary, is intended to cover modifications and equivalent arrangements that are within the spirit and scope of the appended claims. For example, it is to be understood that the present disclosure contemplates that, to the extent possible, one or more features of any embodiment may be combined with one or more features of any other embodiment.

## Claims

1. A tub for holding one or more nests each configured to retain a plurality of medical components, the tub comprising:
a bottom wall;
a first pair of opposed lateral sloped walls and a second pair of opposed lateral sloped walls extending upwardly from the bottom wall and defining an inner volume of the tub, with corners formed between the respective walls of the first pair of opposed lateral sloped walls and the second pair of opposed lateral sloped walls, and with the first pair of opposed lateral sloped walls, the second pair of opposed lateral sloped walls, and the corners including an inner wall surface and an outer wall surface; and
a top flange formed on the first pair of opposed lateral sloped walls and the second pair of opposed lateral sloped walls, with the top flange defining an upper opening of the tub;
wherein each of the first pair of opposed lateral sloped walls and the second pair of opposed lateral sloped walls comprises a stepped wall including a lower wall portion and an upper wall portion, with a ledge formed between the lower wall portion and the upper wall portion and including an inner surface and an outer surface;
**characterized in that** each corner comprises a corner rib extending inwardly from the inner surface thereof, each corner rib extending vertically upwardly from the ledge to a location that is vertically below the top flange.

2. The tub of claim 1, wherein the corner ribs provide for stacking of the tub with another tub of identical construction, the tubs comprising a lower tub and an upper tub, with a top edge of each of the corner ribs on the lower tub engaging the outer surface of the ledge on the upper tub, to support the upper tub on the lower tub and partially nest the upper tub within the lower tub.

3. The tub of claim 2, wherein the ledge extends outwardly from a top edge of the lower wall portion to a bottom edge of the upper wall portion, with the corner guides extending upwardly from the inner surface of ledge.

4. The tub of claim 3, wherein the ledge is parallel to the bottom wall and is configured to support a bottom-most nest of the one or more nests held in the tub.

5. The tub of any of claims 2-4, wherein each of the first pair of opposed lateral sloped walls, each of the second pair of opposed lateral sloped walls, or both each of the first pair of opposed lateral sloped walls and each of the second pair of opposed lateral sloped walls, includes one or more guide features thereon extending upwardly from the ledge, with each of the one or more guide features defining a projection on the inner wall surface and a recess on the outer wall surface.

6. The tub of claim 5, wherein each of the guide features includes a chamfered top surface that is inset inwardly and downwardly from the top flange.

7. The tub of claim 5 or claim 6, wherein the recess of each respective guide feature of the upper tub slides down into engagement with the projection of each respective guide feature of the lower tub, to guide the upper tub into stacking engagement with the lower tub.

8. The tub of claim 7, wherein with the upper tub partially nested within the lower tub, a height of the stacked upper tub and lower tub is approximately 1.5x a height of one of the upper tub or the lower tub.

9. The tub of claim 8, wherein the tub is configured to hold the one or more nests therein, with each of the one or more nests comprising a plurality of chimneys configured to retain syringe stoppers therein.

10. A tub for holding one or more nests each configured to retain a plurality of medical components, the tub comprising:
a bottom wall;
a first pair of opposed lateral sloped walls and a second pair of opposed lateral sloped walls extending upwardly from the bottom wall and defining an inner volume of the tub, with the first pair of opposed lateral sloped walls and the second pair of opposed lateral sloped walls including an inner wall surface and an outer wall surface; and
a top flange formed on the first pair of opposed lateral sloped walls and the second pair of opposed lateral sloped walls, with the top flange defining an upper opening of the tub;
wherein each of the first pair of opposed lateral sloped walls and the second pair of opposed lateral sloped walls comprises a stepped wall including a lower wall portion and an upper wall portion, with a ledge formed between the lower wall portion and the upper wall portion; and
wherein each of the first pair of opposed lateral sloped walls, each of the second pair of opposed lateral sloped walls, or both each of the first pair of opposed lateral sloped walls and each of the second pair of opposed lateral sloped walls, includes one or more guide features thereon extending upwardly from the ledge, with each of the one or more guide features defining a projection on the inner wall surface and a recess on the outer wall surface;
**characterized in that** the each of the one or more guide features includes a rib positioned within the recess that extends from a top end of the recess to a bottom end of the recess.

11. The tub of claim 10, wherein the guide features provide for stacking of the tub with another tub of identical construction, the tub comprising an upper tub and the another tub comprising a lower tub, with a bottom edge of each of the ribs on the upper tub engaging the lower tub, to support the upper tub on the lower tub and partially nest the upper tub with the lower tub.

12. The tub of claim 11, wherein the bottom edge of each of the ribs on the upper tub engages a top surface of a respective projection on the lower tub, to support the upper tub on the lower tub and partially nest the upper tub with the lower tub.

13. The tub of claim 12, wherein the projection of each respective guide pillar extends up to the top flange, with the top surface comprising a flat surface extending inwardly from the top flange.

14. The tub of claim 12, wherein the top surface of each respective guide pillar comprises a chamfered top surface that is inset inwardly and downwardly from the top flange.

15. The tub of claim 14, wherein the chamfered top surface comprises an angled top surface that slopes inwardly and downwardly, and wherein the bottom edge of each of the ribs comprises an angled bottom surface that mates with the angled top surface.
